# EUROPEAN PATENT APPLICATION

(11) **EP 4 148 126 A1**
(43) Date of publication of application: **15.03.2023**
(21) Application number: 21775600.6
(22) Date of filing: 22.03.2021
(51) Int. Cl.: C12N 7/00, C07K 14/00, A61K 35/761, A61P 35/00

(54) **IMMUNOEVASIVE ANTI-TUMOR ADENOVIRUS**

(30) Priority: 25.03.2020 KR 20200036410
(71) Applicant: Curigin Co.,Ltd., Seoul 04778 (KR)
(72) Inventor: CHOI, Jin-Woo, Seongnam-si Gyeonggi-do 13559 (KR); PARK, Seong-Hoon, Wanju-gun Jeollabuk-do 55363 (KR); PETER CHARLES, Goughnour, Suwon-si Gyeonggi-do 16692 (KR); YOO, Jung-Ki, Yangju-si Gyeonggi-do 11456 (KR); CHOI, Chung-Gab, Incheon 21596 (KR); UM, Ki-Hwan, Ansan-si Gyeonggi-do 15338 (KR); LEE, Hyung-Been, Seoul 04779 (KR)
(74) Representative: BCKIP Part mbB
(86) International application number: PCT/KR2021/003491
(87) International publication number: WO 2021/194183

(57) **Abstract**

The present invention relates to an anti-tumor adenovirus that can evade the *in-vivo* immune system. The adenovirus having high ability to kill tumor of the present invention, by comprising a nucleic acid encoding a tumor albumin-binding domain, has significantly increased effects of infecting and killing tumor cells, exhibits an increase in binding with albumin to thereby evade *in-vivo* immune responses, leading to an increase in plasma half-life, is specifically delivered to cancer cells to produce systemic therapeutic effects, and can be topically delivered and has excellent selectivity, resulting in a remarkable effect in anti-tumor efficacy and, therefore, the adenovirus can be advantageously used as an anticancer composition or anticancer adjuvant in various types of cancer.

## Description

### [Technical Field]

The present invention relates to an anti-tumor adenovirus capable of avoiding an *in vivo* immune system.

### [Background Art]

Cancer is one of the most fatal diseases in the world, and the development of an innovative therapeutic agent for cancer can reduce medical costs generated during treatment thereof and create high-added value at the same time. In addition, according to the statistics in 2008, molecular therapeutic agents capable of overcoming resistance to existing anticancer drugs accounted for $17.5 billion in seven major countries (US, Japan, France, Germany, Italy, Spain, UK), and in 2018, it is expected to show a growth rate of 9.5% compared to 2008, accounting for about $45 billion in market size. The treatment of cancer is classified into surgery, radiation therapy, chemotherapy, and biological therapy, and among these treatments of cancer, chemotherapy is a treatment that suppresses or kills the proliferation of cancer cells as a chemical material, and the toxicity shown by an anticancer agent is also largely shown in normal cells, so that it shows a certain degree of toxicity; however, even though the anticancer agent shows an effect, resistance occurs in which the effect is lost after a certain period of use, and thus, it is urgent to develop an anticancer agent that selectively acts on cancer cells and does not produce resistance (the present address of cancer conquest, Biowave 2004.6(19)). Recently, the development of new anticancer drugs targeting molecular characteristics of cancer has been made by securing the information on the molecular distribution of cancer, and there are reports that anticancer drugs targeting specific molecular targets that only cancer cells have do not exhibit drug resistance.

Due to unproven reports of a transient cancer remission following natural viral infection or viral vaccination, there have been experiments using the virus for cancer treatment. The reduction of cervical cancer in patients vaccinated for rabies was first reported in 1912, and similar results were seen in cancer patients with smallpox vaccination or a natural viral infection such as mumps or measles. It was in the late 1940s and early 1950s that patients were inoculated with live viruses for cancer treatment based on these reports and animal data. However, sometimes after temporary tumor reduction, problems of tumor re-growth and patient death occurred. These inoculations showed no long-lasting remission. In 1957, Albert B. Sabin, M.D., who developed the live oral polio vaccine, noted that even when the oncolytic virus kills a tumor, the biggest problem is that the individual's immune response to the virus is so fast that its effects are rapidly exhausted.

At this point, a number of oncolytic viruses have been identified, but the only virus approved for clinical use anywhere in the world to date is the Oncorine (H101) subgroup C adenovirus modified by the E1B-55KD deletion that allows conditional replication in P53-deficient cancer cells (H101 is a close analog of ONYX015 described by Bischoff *et al.* in 1996). Oncorine is administered by intra-tumor injection for head and neck cancer. Adenoviruses have been widely used not only as gene transfer vectors for gene therapy, but also as oncolytic agents for the treatment of cancer. Adenovirus exhibits several characteristics suitable for this use. In other words, the structure and biological properties of adenovirus have been widely studied, and thus their genome can be easily modified, and these viruses can infect both replicable and nonreplicable cells, and can be easily produced with high titers for clinical use. Adenoviruses, from a safety standpoint, do not cause life-threatening diseases in humans, and their viral genome is non-integrative, in which insertion mutations are prevented. Clinical trials using adenovirusbased vectors have reported that the virus has good toxicity and safety profiles, although the need to improve its efficacy remains for systemic administration.

As such, in the field of gene therapy, systemic administration, i.e., injection into intravenous or intraarterial blood flow, may be required to reach multiple organs and disseminated cells. For example, systemic administration is essential to treat disseminated tumors in advanced or metastatic states in cancer therapy using adenoviral vectors and oncolytic adenoviruses. However, adenovirus shows a significant limitation in lowering the therapy effect upon injection into the bloodstream. Adenovirus type 5 (Ad5) undergoes various neutralizing interactions that sharply decrease the bioavailability of the virus in the bloodstream. Since more than 90% of the injected dose resides in the liver, liver macrophages, which are often referred to as Cooper cells, and also in liver LSEC (liver sinusoidal endothelial cell) and liver cells, the biggest problem with this therapy is liver isolation. Direct interaction with blood cells and proteins is also a major obstacle. Ad5 binds directly to blood cells such as red blood cells through the CAR receptor and can bind to platelets through integrin. Antibodies may not only neutralize virus directly, but also trigger innate immune responses by complement activation and docking of virus particles with monocytes and Fc receptors in neutrophils. In addition, virus readministration increases the concentration of anti-Ad neutralizing antibodies (NAb), and thus virus neutralization is also enhanced. Opsonization of adenovirus by antibodies and complement can also enhance clearance by Cooper cells. Overall, these interactions result in greatly shortening the half-life of Ad in blood to about several minutes in mice and humans. Although considerable efforts have been made to avoid neutralization by antibodies and immune cells upon systemic administration of adenovirus, it has been reported that systemic transmission is still limited, and that it is temporary and usually ineffective (Ferguson *et al.,* 2012).

Therefore, there is still a need for research on adenovirus which is suitable for systemic administration and is a cancer therapeutic agent capable of avoiding neutralizing antibodies.

### [Disclosure]

### [Technical Problem]

An object of the present invention is to provide an anti-tumor adenovirus.

Furthermore, an object of the present invention is to provide a composition for treating cancer.

### [Technical Solution]

In order to solve the above problem, the present invention provides an anti-tumor adenovirus including a nucleic acid encoding an albumin binding domain.

In addition, the present invention provides a composition for treating cancer, including the anti-tumor adenovirus.

### [Advantageous Effects]

According to the present invention, the adenovirus having a high tumor cell killing ability of the present invention includes a nucleic acid encoding a tumor albumin binding domain, thereby remarkably increasing effects of infecting and killing tumor cells, and increasing binding to albumin, thereby avoiding *in vivo* immune responses, increasing plasma half-life, specifically delivered to cancer cells, having systemic therapeutic effects, enabling local delivery, showing remarkable anti-tumor efficacy due to excellent selectivity, and can be useful as an anticancer composition or an anticancer adjuvant for various carcinomas.

### [Description of Drawings]

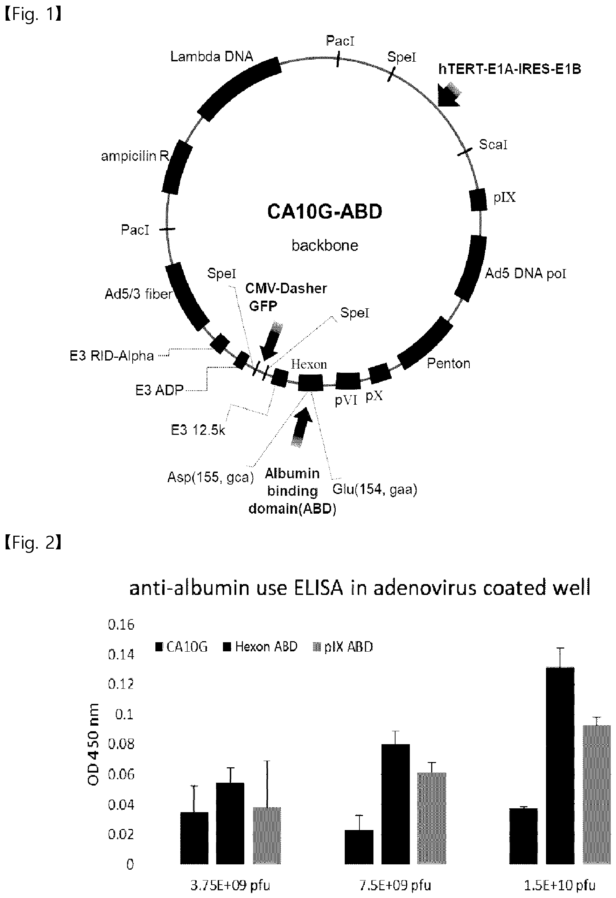

FIG. 1 is a diagram illustrating a vector map of adenovirus including an albumin binding domain of the present invention.
FIG. 2 is a diagram showing the albumin binding ability of the adenovirus (Hexon and pIX) of the present invention including an albumin binding domain: CA10G: control adenovirus; Hexon ABD: adenovirus including an albumin binding domain in Hexon of an adenovirus vector; and pIX ABD: adenovirus including an albumin binding domain in pIX of an adenovirus vector.

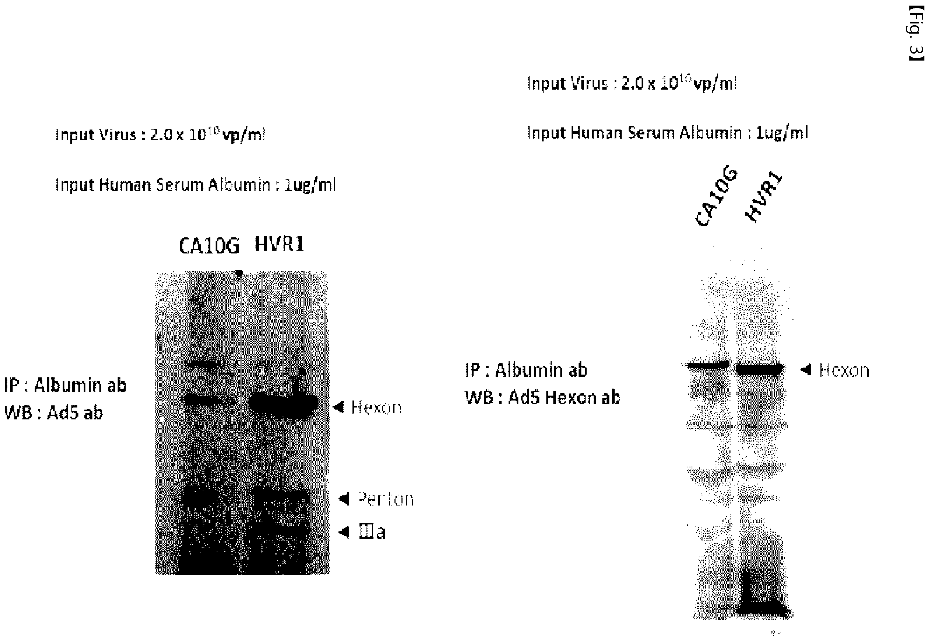

FIG. 3 is a graph comparing the binding ability of the conventional anti-tumor virus CA10G, which does not include an albumin binding domain, and human albumin of the anti-tumor virus of the present invention, which includes an albumin binding domain in the Hexon region, through IP analysis: CA10G: control anti-tumor adenovirus; and HVR1: adenovirus including an albumin binding domain in Hexon of an adenovirus vector.

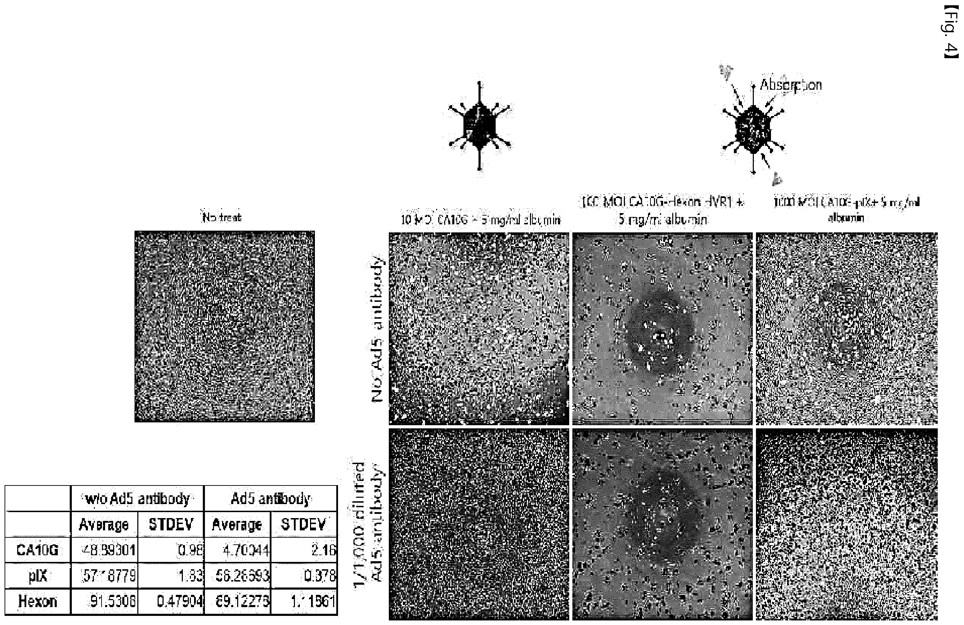

FIG. 4 is an image showing the tumor cell killing effect by albumin binding of the adenovirus (Hexon and pIX) of the present invention including an albumin binding domain: CA10G: control adenovirus; CA10G-Hexon: adenovirus including an albumin binding domain in Hexon of an adenovirus vector; and CA10G-pIX: adenovirus including an albumin binding domain in pIX of an adenovirus vector.

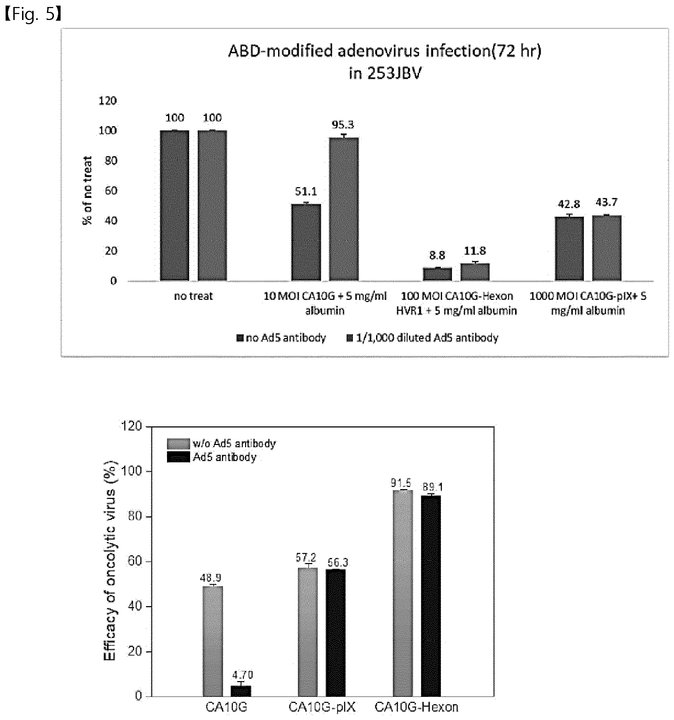

FIG. 5 is a diagram quantifying the killing (top) and infection (bottom) effects of bladder cancer cells by albumin binding of the adenovirus (CA10G-Hexon and CA10G-pIX) of the present invention including an albumin binding domain.

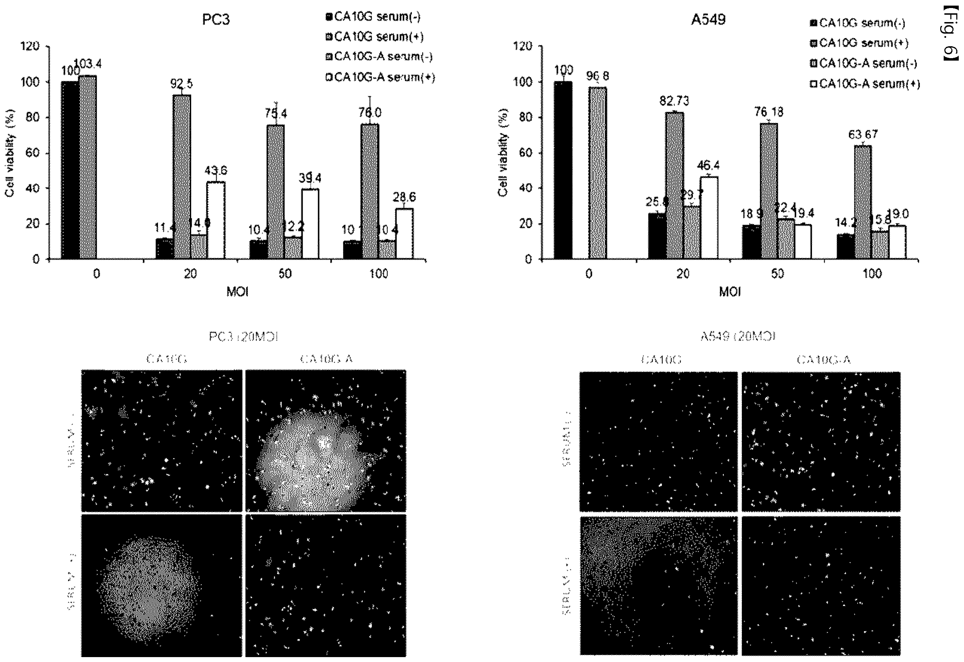

FIG. 6 is a diagram quantifying the killing (top) and infection (bottom) effects of prostate cancer cells and lung cancer cells by albumin binding of an adenovirus of the present invention including an albumin binding domain: CA10G: a control adenovirus; and CA10G-A: adenovirus including an albumin binding domain in Hexon of an adenovirus vector.

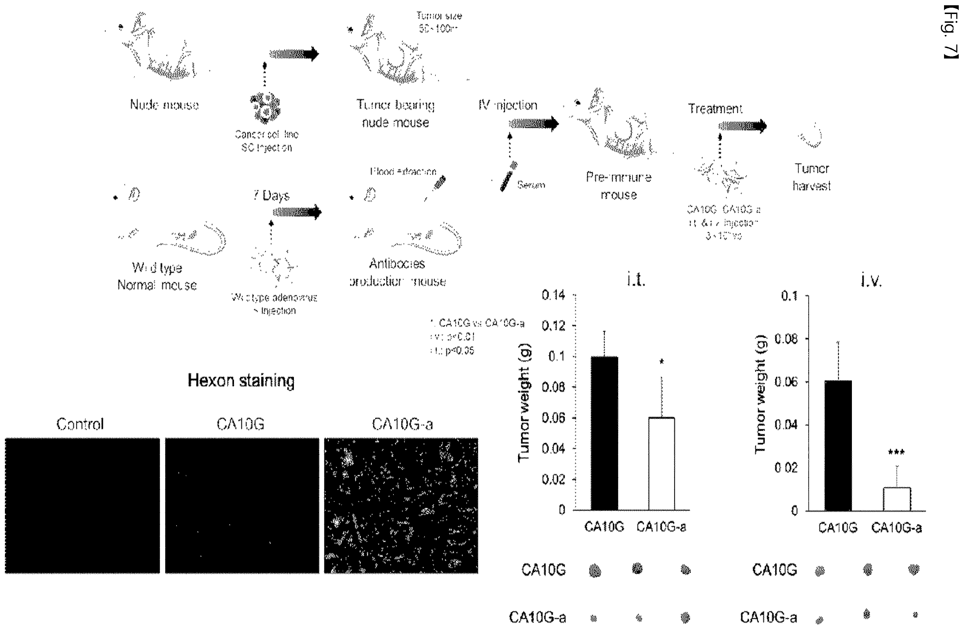

FIG. 7 is a diagram showing the neutralizing antibody avoidance ability (Hexon staining) of the adenovirus of the present invention and the effect of reducing the size and weight of the tumor by the same, which were confirmed by *in vivo:* i.v.: intravenous injection; i.t.: intratumoral injection; CA10G: control adenovirus; and CA10G-a: adenovirus including an albumin binding domain in Hexon of an adenovirus vector.

### [Modes of the Invention]

Hereinafter, the present invention will be described in detail as an embodiment of the present invention. However, the following embodiments are presented as examples of the present invention, and the present invention is not limited thereto, and the present invention may be variously modified and applied within the description of the following claims and the equivalents interpreted therefrom.

Unless otherwise indicated, nucleic acids are recorded in the 5'→3' direction from left to right. Numerical ranges listed within the specification include numbers defining the range, and include each integer or any non-integer fraction within the defined range.

Unless otherwise defined, all technical and scientific terms used herein have the same meanings as those commonly understood by those skilled in the art to which the present invention pertains. While any method and materials similar or equivalent to those described herein can be used in the practice for testing the present invention, preferred materials and methods are described herein.

In one aspect, the present invention relates to an anti-tumor adenovirus including a nucleic acid encoding an albumin binding domain (ABD).

In an embodiment, the nucleic acid encoding the albumin binding domain may be included in the coding region of the Hexon of adenovirus, and the Hexon may include the base sequence represented by SEQ ID NO: 3. In the present invention, a virus including a nucleic acid encoding an albumin binding domain in the coding region of Hexon of the adenovirus vector CA10G, which is an anti-cancer (anti-tumor) virus, may be used interchangeably as CA10G-Hexon, HVR1, CA10G-a, or Hexon ABD.

In an embodiment, a nucleic acid encoding an albumin binding domain may be included between the 154th amino acid (gaa, Glu) and the 155th amino acid (gca, Asp) of the Hexon protein encoded by the base sequence represented by SEQ ID NO: 3, and the base sequence of the Hexon containing ABD may include the base sequence represented by SEQ ID NO: 6.

In an embodiment, the nucleic acid encoding the albumin binding domain may be included in a base sequence encoding pIX (protein IX) of adenovirus, and more preferably, the nucleic acid may be included at the C-terminus of pIX, the base sequence encoding pIX may include a base sequence represented by SEQ ID NO: 13, and the base sequence encoding a protein having an albumin binding domain at the C-terminus of pIX may include a base sequence represented by SEQ ID NO: 14.

In an embodiment, the nucleic acid encoding the albumin binding domain may include a base sequence represented by SEQ ID NO: 1.

In an embodiment, the albumin binding domain may include an amino acid sequence represented by SEQ ID NO: 2.

In one embodiment, the anti-tumor adenovirus of the present invention may further include a human telomere promoter (hTERT) (SEQ ID NO: 7).

In one embodiment, the human telomere promoter can be operatively linked to an endogenous gene of an adenovirus, in which the endogenous gene of the adenovirus has the structure 5'ITR-C1-C2-C3-C4-C5 3'ITR; in which the C1 includes E1A (SEQ ID NO: 8), E1B (SEQ ID NO: 9), or E1A-E1B; in which the C2 includes E2B-L1-L2-L3-E2A-L4; in which the C3 does not include E3 or includes E3; in which the C4 includes L5; and in which the C5 does not include E4 or includes E4.

In the present invention, the term "operatively linked" refers to a functional binding between a gene expression regulatory sequence (e.g., a promoter, a signal sequence, or an array of transcription regulatory factor binding sites) and another gene sequence, whereby the regulatory sequence controls transcription and/or translation of the other gene sequence.

In one embodiment, the hTERT promoter can be operatively linked to E1A and E1B of the endogenous genes of an adenovirus.

In one embodiment, the IRES sequence (SEQ ID NO: 10) may be further included between E1A and E1B.

In one embodiment, the anti-tumor adenovirus of the present invention may further include an hTERT promoter-E1A-IRES-E1B sequence including the base sequence represented by SEQ ID NO: 4.

The adenoviruses of the present invention can be selectively distributed *in vivo* in a given tissue, avoiding or significantly reducing expression in non-target or non-tumor tissue.

The replicative adenovirus of the present invention may have modifications in its genomic sequence that confer selective replication in a cell. In order to apply the expression of the adenovirus to the tissue in which such expression is needed or to the tumoral tissue to be treated, the adenovirus of the present invention may include a tissue-specific promoter or a tumor-specific promoter. Thus, in an embodiment, the adenovirus further includes a tissue-specific promoter or a tumor-specific promoter.

The term "promoter", as used herein, is used according to its art-recognized meaning. It is intended to mean the DNA region, usually located upstream to the coding sequence of a gene, which binds RNA polymerase and directs the enzyme to the correct transcriptional start site. Said promoter controls the viral genes that start the replication.

In the present invention, the term "tissue-specific" is intended to mean that the promoter to which the gene essential for replication is operably linked functions specifically in that tissue so that replication proceeds in that tissue. This can occur by the presence in that tissue, and not in non-target tissues, of positive transcription factors that activate the promoter. It can also occur by the absence of transcription inhibiting factors that normally occur in non-target tissues and prevent transcription as a result of the promoter. Thus, when transcription occurs, it proceeds into the gene essential for replication such that in a target tissue, replication of the vector and its attendant functions occur. Tissue specificity is particularly associated with targeting abnormal counterparts of a particular tissue while avoiding normal counterparts of the tissue, or avoiding peripheral tissues of other types other than abnormal tissue while treating the abnormal tissue. In certain embodiments, the promoter is "tumor-specific," meaning that the promoter functions specifically in tumor tissue.

In one embodiment, the expression cassette expressing the foreign gene of the present invention may be further included, and the expression cassette expressing the foreign gene may be included in the E3 region of the endogenous gene of the adenovirus.

In one embodiment, the anti-tumor adenovirus of the present invention may further include a CMV promoter (SEQ ID NO: 11) and a GFP encoding nucleic acid (SEQ ID NO: 12), may include the base sequence represented by SEQ ID NO: 5, and may include a CMV promoter and a GFP encoding nucleic acid at the E3 region of the endogenous gene of the adenovirus.

In one embodiment, the anti-tumor adenovirus of the present invention may be selected from the group consisting of human adenovirus serotypes 1 to 57.

In one embodiment, the anti-tumor adenovirus of the present invention may be of human adenovirus serotype 5. The gene sequence of human adenovirus serotype 5 can be found in GenBank: AY339865.1 (version of 13th August 2007).

In one embodiment, the anti-tumor adenovirus of the present invention may have higher tumor cell killing ability than wild-type adenovirus, and may be a oncolytic adenovirus.

In one embodiment, the anti-tumor adenovirus of the present invention may be used for cancer gene treatment.

In the present invention, the term "promoter" refers to an untranslated nucleic acid sequence upstream of the coding region, which includes a binding site for RNA polymerase and has the activity of initiating transcription of a gene downstream of the promoter into mRNA. In the expression cassette of the present invention, the promoter may be any promoter capable of initiating the expression of shRNA. Specifically, the promoter of the present invention may be a constitutive promoter which induces the expression of a target gene at all times or an inducible promoter which induces the expression of a target gene at a specific position and time, and examples thereof are a U6 promoter, an H1 promoter, a cytomegalovirus (CMV) promoter, an SV40 promoter, a CAG promoter (Hitoshi Niwa et al., Gene, 108: 193-199, 1991), a CaMV 35S promoter (Odell et al., Nature 313: 810-812, 1985), a Rsyn7 promoter (U.S. Patent Application No. 08/991,601), a rice actin promoter (McElroy et al., Plant Cell 2: 163-171, 1990), a ubiquitin promoter (Christensen et al, Plant Mol. Biol. 12:619-632, 1989), an ALS promoter (U.S. Patent Application No. 08/409,297), and the like. In addition, all known promoters apparent to those skilled in the art, such as the promoters disclosed in U.S. Pat. Nos. 5,608,149; 5,608,144, 5,604,121, 5,569,597, 5,466,785, 5,399,680, 5,268,463, 5,608,142, etc. may be used, but are not limited thereto. Preferably, the promoter of the present invention may be a U6 promoter, an HI promoter, or a CMV promoter, and according to a preferred embodiment of the present invention, the CMV promoter may be used.

According to the present invention, the term "adenovirus" refers to any virus that can be categorized as an adenovirus, i.e., any virus pertaining to the Adenoviridae family characterized by being a non-enveloped virus with an icosahedral nucleocapsid containing a double-stranded DNA genome. This term includes any adenovirus capable of infecting a human or an animal, including all groups, subgroups, and serotypes that use CAR as a receptor for infection of target cells. Adenoviruses of the present invention include, without limitation, avian, canine, equine, bovine, ovine, porcine, human, or frog adenovirus. In a preferred embodiment, the adenovirus of the present invention is a human adenovirus, i.e., an adenovirus capable of infecting humans. According to the present invention, a "serotype" is each of the immunologically different types of adenoviruses. There are at least 57 serotypes of human adenovirus that are classified into several subgroups (A to G).

The adenovirus of the present invention is a recombinant adenovirus. According to the present invention, the term "recombinant" refers to an adenovirus that does not appear naturally. This recombinant adenovirus contains one or more modifications with respect to the wild-type. Such modifications include, but are not limited to, modifications to the adenovirus genome that is packaged in the particle in order to make an infectious virus. Other modifications allow obtaining replication-deficient virus (i.e., virus that cannot reproduce) by removing a gene that is critical for replication, from the virus genome. Exemplary modifications include deletions known in the art, such as deletions in one or more of the E1a, E1b, E2a, E2b, E3, or E4 coding regions. Other modifications include deletions of all of the coding regions of the adenoviral genome. Such adenoviruses are known as "gutless" adenoviruses. Chimeric adenoviruses formed by a combination of elements from different serotypes are also included.

According to the present invention, the term "recombinant" also includes replicationconditional adenoviruses, which are viruses that preferentially replicate in certain types of cells or tissues but to a lesser degree or not at all in other types. For example, among the adenoviruses provided herein are adenoviruses that replicate in abnormally proliferating tissue, such as solid tumors and other neoplasms. These include the viruses disclosed in U.S. Pat. Nos. 5,998,205 and 5,801,029. Such viruses are sometimes referred to as "cytolytic" or "cytopathic" viruses (or vectors), and, if they have such an effect on neoplastic cells, are referred to as "oncolytic" viruses (or vectors).

In the present invention, the term "adenoviral Hexon protein" or "Hexon protein" (formerly referred to as "protein II") refers to the major structural capsid protein found in adenoviruses that self-associates to form trimers, each in the shape of a hexagon. 240 Hexon trimers are assembled to provide an adenoviral capsid. The Hexon protein is essential for virus capsid assembly, determination of the icosahedral symmetry of the capsid, and integrity of the capsid. The major structural features of the Hexon protein are common among adenovirus serotypes, but the Hexon protein differs in size and immunological properties between serotypes.

In the present invention, the term "Hexon protein" encompasses the Hexon protein of any adenovirus, including, without limitation, the protein defined by the sequence of the UniProt database with accession number P04133 dated 19 Feb. 2014 which corresponds to the Hexon protein of human adenovirus C serotype 5; the protein defined by the sequence of the UniProt database with accession number P03277 dated 19 Feb. 2014 which corresponds to the Hexon protein of human adenovirus C serotype 2; the protein defined by the sequence of the UniProt database with accession number P42671 dated 19 Feb. 2014 which corresponds to the Hexon protein of avian adenovirus gall (strain Phelps); and the protein defined by the sequence of the UniProt database with accession number P11819 dated 19 Feb. 2014 which corresponds to the Hexon protein of human adenovirus F serotype 40. The expression includes all the natural variants of Hexon protein that appear naturally in other subgroups or serotypes. It has been reported that Loop 1 (L1) and Loop 2 (L2) of the Hexon protein are exposed on the outside surface of the virus capsomere structure.

In the present invention, the term "albumin" refers to a member of the albumin family proteins that are water-soluble globular proteins, moderately soluble in concentrated salt solutions, and experiencing heat denaturation. Albumins are commonly found in blood plasma. Serum albumin is produced by the liver, is dissolved in blood plasma, and is the most abundant blood protein in mammals.

In the present invention, the term "albumin binding domain" refers to any region from a naturally occurring protein which is capable of binding albumin with sufficient specificity so as to ensure protection from neutralizing antibodies.

The present invention includes a functionally equivalent variant of the albumin binding domain described above. In the present invention, the term "functionally equivalent variant" refers to any polypeptide that is derived from the albumin binding domain by insertion, deletion, or substitution of one or more residues and that substantially retains the ability to interact with albumin as determined above. In a preferred embodiment, a polypeptide is considered a functionally equivalent variant of an albumin binding domain if it exhibits an albumin binding capacity of at least 10%, 20%, 30%, 40%, 50%, 60%, 70%, 80%, 90%, or 100% of the albumin binding capacity of the albumin binding domain represented by SEQ ID NO: 2. Preferably, a polypeptide is considered a functionally equivalent variant of an albumin binding domain if it is capable of neutralizing an antibody with an efficiency of at least 10%, 20%, 30%, 40%, 50%, 60%, 70%, 80%, 90%, or 100% with the albumin binding domain represented by SEQ ID NO: 2. Suitable functional variants are those having at least 25% amino acid sequence identity, e.g., at least 30%, at least 40%, at least 50%, at least 60%, at least 70%, at least 80%, at least 90%, at least 91%, at least 92%, at least 93%, at least 94%, at least 95%, at least 96%, at least 97%, at least 98%, or at least 99% identity, with respect to the albumin binding domain sequence disclosed herein. The level of identity between the two polypeptides is determined using computer algorithms and methods well known to those skilled in the art. The identity between the two amino acid sequences is preferably determined using a BLASTP algorithm [BLAST Manual, Altschul, S., et al., NCBI NLM NIH Bethesda, Md. 20894, Altschul, S., et al., J. Mol. Biol. 215: 403-410 (1990)], other similar algorithms may also be used. The BLAST and BLAST 2.0 are used to determine % sequence identity using the parameters mentioned herein. Software for performing BLAST analysis is publicly available through the National Center for Biotechnology Information.

Recombinant adenovirus can be obtained through standard molecular biology techniques known in the art (Chillon and Bosch. Adenovirus. Methods and Protocols. 3rd edition. Methods in Molecular Biology, vol. 1089. Springer Protocols. Humana Press. (2014)). The adenoviruses including the albumin binding domain of the present invention are as referred in Chillon and Bosch Adenovirus. Methods and Protocols. 3rd edition. Methods in Molecular Biology, vol. 1089. Springer Protocols. Humana Press. (2014); and Alemany R, Zhang W. Oncolytic adenoviral vectors. Totowa, NJ.: Humana Press, 1999, it is amplified and proliferated according to standard methods in the field of adenoviral vectors. Cell lines commonly used in the field of gene therapy and virotherapy are the HEK-293 cell line and the A549 cell line. A preferred proliferation method is to infect a cell line that allows replication of adenovirus. The lung adenocarcinoma A549 cell line is an example of such a cell line. Proliferation is performed, for example, as follows: A549 cells are inoculated into plastic cell culture plates and 100 viral particles per cell are infected. After 2 days, when the cells are desorbed to form clusters "like grapes", the cytopathic action demonstrates virus production. Cells are recovered *in vitro.* After centrifugation at 1000 g for 5 minutes, the cell pellet is frozen and thawed three times to destroy the cells. The obtained cell extract is centrifuged at 1000 g for 5 minutes, and a cesium chloride concentration gradient is laminated on the virus-containing supernatant, and centrifuged at 35000 g for 1 hour. The viral bands obtained by the concentration gradient are collected and dialyzed against PBS-10% glycerol. The dialyzed virus is isolated and stored at -80°C. The number of viral particles and plaque-forming units are quantified according to standard protocols. Phosphate buffered saline (PBS) with 5% glycerol added is a standard formulation used to store adenovirus. However, other formulations that improve the stability of the virus are also disclosed.

The method for purifying an adenovirus including an albumin binding domain for use in cancer prevention or treatment is equally applied to other adenovirus and adenovirus vectors used for cancer virotherapy and gene therapy.

In one aspect, the present invention relates to a composition for treating cancer, including the anti-tumor adenovirus of the present invention and a pharmaceutically acceptable carrier.

In one embodiment, the composition of the present invention may be administered systemically.

In one embodiment, the composition of the present invention may be for intravenous administration.

In one embodiment, the composition of the present invention may further include an anticancer agent, for example, acivicin, aclarubicin, acodazole, acronicin, azogelesin, alanosine, aldesleukin, allopurinol sodium, altretamine, aminoglutetimide, ammonafide, ampligen, amsacrine, androgens, anguidine, apidicoline glycinate, asaray, asparaginase, 5-azacytidine, azathioprine, Bacillus calmete-guerein (BCG), Bakers antipol, Beta-2-dioxythioguanosine, bisanthrene HCl, bleomycin sulfate, bullserpan, buthionine sulfoximine, BWA 773U82, BW 502U83/HCl, BW 7U85 mesylate, cerasemide, carbetimer, carboplatin, carmustine, chlorambucil, chloroquinoxaline-sulfonamide, chlorozotocin, chromomycin A3, cisplatin, cladribine, corticosteroids, corynerbacterium parvum, CPT-11, crisnatol, cyclocytidine, cyclophosphamide, cytarabine, cytembena, davis maleate, deccarbazine, dactinomycin, daunorubicin HCl, diaziridine, dexrazoxane, dianhydride galactitol, diazicuone, dibromodulcitol, didemine B, diethyldithiocarbamate, diclycoaldehyde, dihydro-5-azacytine, doxorubicin, echinomycin, dedatrexate, edelphosine, eplonitine, elliot solution, elsamitrucin, epirubicin, esorubicin, estramustine phosphate, estrogen, ethanidazole, ethioforce, etoposide, padrazol, parazabine, phenretinide, filgrastim, pinasteride, flavone acetic acid, floxuridine, fludarabine phosphate, 5'-fluorouracil, Fluosol^{™}, flutamide, gallium nitrate, gemcitabine, goserelin acetate, hefsulfam, hexamethylene bisacetamide, homoharringtonine, hydrazine sulfate, 4-hydroxyandrostenedione, hydroziurea, idarubicin HCl, ifosfamide, 4-ipoeanol, iproplatin, isotretinoin, leucovorin calcium, leuprolide acetate, levamisole, liposome daunorubicin, liposome capture doxorubicin, romustine, ronidamine, maytansine, mechlorethamine hydrochloride, melphalan, menogalyl, merbarone, 6-mercaptopurine, mesena, methanol extracts of bacillu calmette-guerin, methotrexate, N-methylformamide, mifepristone, mitoguazone, mitomycin-C, mitotan, mitoxantrone hydrochloride, monocyte/macrophage colony-stimulating factor, nabilone, napoxidine, neocarzinostatin, octreotide acetate, ormaplatin, oxaliplatin, paclitaxel, pala, pentostatin, piperazinedione, fiforbroman, pirarubicin, piritrexim, pyroxantrone hydrochloride, PIXY-321, plicamycin, porfimer sodium, prednimustine, procarbazine, progestin, pyrazofurin, razoxane, sargramostim, semustine, spirogermanium, spiromustine, streptonigrin, streptozocin, sulophener, suramin sodium, tamoxifen, taxorere, tegafur, Teniposide, Terephthalamidine, Terroxirone, Thioguanine, Thiotepa, Thymidine Injection, Thiazopurine, Topotecan, Toremifene, Tretinoin, Trifluoperazine hydrochloride, Trifluridine, Trimetrexate, TNF (tumor necrosis factor), uracil mustard, vinblastine sulfate, vincristine sulfate, vindesine, vinorelbine, vincolidine, Yoshi 864, Zorubicin, cytosine arabinoside, etoposide, melphalan, taxol, and mixtures thereof. Preferably, it is cisplatin, paclitaxel, 5-FU (5-fluorouracil), methotrexate, doxorubicin, daunorubicin, cytosine arabinoside, etoposide, melphalan, chlorambucil, cyclophosphamide, vindesine, mitomycin, bleomycin, tamoxifen, and taxol, and more preferably cisplatin, paclitaxel, or 5-FU (5-fluorouracil), but the present invention is not limited thereto, so long as it is used in combination with the composition of the present invention to achieve the purpose of synergistic effects on anticancer effects.

In one embodiment, the cancer may be any one selected from the group consisting of colon cancer, breast cancer, uterine cancer, cervical cancer, ovarian cancer, prostate cancer, brain tumor, head and neck carcinoma, melanoma, myeloma, leukemia, lymphoma, gastric cancer, lung cancer, pancreatic cancer, non-small cell lung cancer, liver cancer, esophageal cancer, small intestine cancer, anal cancer, fallopian tube carcinoma, endometrial carcinoma, vaginal carcinoma, vulval carcinoma, Hodgkin's disease, bladder cancer, kidney cancer, ureter cancer, renal cell carcinoma, renal pelvic carcinoma, bone cancer, skin cancer, head cancer, neck cancer, skin melanoma, intraocular melanoma, endocrine gland cancer, thyroid cancer, parathyroid cancer, adrenal cancer, soft tissue sarcoma, urethral cancer, penile cancer, central nervous system (CNS) tumor, primary CNS lymphoma, spinal tumor, glioblastoma, and pituitary adenoma.

The composition of the present invention may further include an adjuvant. Any one of the adjuvants known in the art may be used without limitation, but for example, the adjuvant may further include a complete adjuvant or an incomplete adjuvant of Freund to increase the effects thereof.

The composition according to the present invention may be prepared in a form in which an active ingredient is incorporated in a pharmaceutically acceptable carrier. Here, the pharmaceutically acceptable carrier includes a carrier, excipient, and diluent commonly used in the pharmaceutical field. Pharmaceutically acceptable carriers that can be used in the compositions of the present invention include, but are not limited to, lactose, dextrose, sucrose, sorbitol, mannitol, xylitol, erythritol, maltitol, starch, acacia rubber, alginate, gelatin, calcium phosphate, calcium silicate, cellulose, methyl cellulose, polyvinyl pyrrolidone, water, methylhydroxybenzoate, propylhydroxybenzoate, talc, magnesium stearate, and mineral oil.

The composition of the present invention may be formulated and used in the form of oral formulations such as powders, granules, tablets, capsules, suspensions, emulsions, syrups, aerosols, etc., external preparations, suppositories, or sterile injectable solutions, respectively, according to conventional methods.

When formulated, a diluent or excipient such as a filler, a bulking agent, a binder, a wetting agent, a disintegrating agent, a surfactant, etc., which are commonly used, may be used. Solid formulations for oral administration include tablets, pills, powders, granules, capsules, and the like, and such solid formulations may be prepared by mixing an active ingredient with at least one excipient, for example, starch, calcium carbonate, sucrose, lactose, gelatin, and the like. In addition to simple excipients, lubricants such as magnesium stearate and talc may also be used. Liquid formulations for oral administration include suspensions, solutions, emulsions, syrups, and the like, and various excipients, for example, wetting agents, sweeteners, perfumes, preservatives, and the like, may be included in addition to water and liquid paraffin, which are generally used diluents. Formulations for parenteral administration include sterilized aqueous solutions, water-insoluble solvents, suspensions, emulsions, lyophilized preparations, and suppositories. Examples of the water-insoluble solvent and the suspension may include vegetable oils such as propylene glycol, polyethylene glycol, and olive oil, injectable esters such as ethyl oleate, and the like. As a base for suppositories, witepsol, tween 61, cacao butter, laurin butter, glycerogelatin, etc. may be used.

The product according to the present invention may be administered to a subject by various routes. All modes of administration may be contemplated, for example, oral, intravenous, intramuscular, subcutaneous, or intraperitoneal injection, although intravenous administration is most preferred.

The administration amount of the pharmaceutical composition according to the present invention is selected in consideration of the age, weight, sex, physical condition, etc. of the subject. It is obvious that the concentration of the single domain antibody included in the pharmaceutical composition may be variously selected depending on the subject, and preferably, the concentration is 0.01 to 5, 000 µg/ml in the pharmaceutical composition. When the concentration is less than 0.01 µg/ml, the pharmaceutical activity may not be exhibited, and when the concentration is more than 5,000 µg/ml, the pharmaceutical activity may be toxic to the human body.

The composition of the present invention may be used for preventing or treating cancer and complications thereof and can also be used as an anticancer adjuvant.

The composition of the present invention is administered in a therapeutically effective amount or a pharmaceutically effective amount. The term "pharmaceutically effective amount" refers to an amount sufficient to treat a disease at a reasonable benefit/risk ratio applicable to medical treatment, and an effective dose level may be determined according to factors including subject type and severity, age, sex, activity of drugs, sensitivity to drugs, administration time, route of administration and rate of excretion, duration of treatment, concurrent drugs, and other factors well known in the medical arts.

In one aspect, the present invention relates to a use of anti-tumor adenovirus including a nucleic acid encoding an albumin binding domain of the present invention for preventing or treating tumors.

In one aspect, the present invention relates to a method for treating a tumor with an anti-tumor adenovirus including a nucleic acid encoding an albumin binding domain of the present invention.

### Best Mode for Carrying Out the Invention

The present invention will be described in more detail with reference to the following examples. However, the following Examples are only for specifying the contents of the present invention, and the present invention is not limited thereto.

### Example 1. Generate anti-cancer (anti-tumor) virus including albumin binding domain

### 1-1. Generate adenovirus in which ABD inserted into Hexon

The base sequence (SEQ ID NO: 1) encoding the *Streptococcus sp.* G148-derived albumin binding domain (albumin binding domain, ABD) was inserted between the 154th amino acid (gaa, Glu) and the 155th amino acid (gca, Asp) of the base sequence (SEQ ID NO: 3) encoding the Hexon of the adenovirus vector (base sequence encoding Hexon-ABD-Hexon: SEQ ID NO: 6), the hTERT promoter-E1A-IRES-E1B sequence (SEQ ID NO: 4) was inserted, and the CMV promoter and the Dasher GFP-encoding sequence (SEQ ID NO: 5) were inserted between the SpeI of E3 region (FIG. 1). The sequences of the completed virus vectors were analyzed, and when there was no abnormality, the viral genome was linearized using PacI restriction enzyme, and then the virus was transduced into 293A cells using CaCl₂ method to produce each virus.

### 1-2. Generate adenovirus in which ABD inserted into Hexon pIX

In this manner, the base sequence (SEQ ID NO: 1) encoding the albumin binding domain was inserted into the C-terminal region of the base sequence (SEQ ID NO: 13) encoding the pIX (protein IX) of adenovirus before the stop codon, TAA (pIX-ABD: SEQ ID NO: 14), thereby producing virus vector to produce virus.

### Example 2. Check binding ability of anti-cancer virus to human albumin

In order to confirm the degree of binding of adenovirus including the albumin binding domain prepared in the above Example to albumin, human albumin ELISA analysis was performed using the virus-coated plate. Specifically, the control virus CA10G, and the aboveprepared pIX-ABD, and Hexon-ABD viruses each were diluted to 1.5 × 10⁹, 7.5 × 10⁸, and 3.75 × 10⁸ pfu/ml with ELISA coating buffer (R&D system, DY006), and then, 100 µl of each virus was added to a 96-well plate and incubated at 4°C overnight. Thereafter, the solution was discarded, and a washing process was performed three times by adding 200 µl of a washing buffer, 5 mg/mL BSA was made by using PBS containing 0.5% Tween20, and the resultant was added to 100 µl/well, and reacted at 37°C for 1 hour. Then, 5 mg/ml of HSA (Sigma, A1653) made of PBS containing 1% BSA after washing 3 times was added to each well at 100 µl, followed by reaction at 37°C for 1 hour. Then, ALB antibodies (Santacruz, sc-271604), which were washed three times and diluted 400 times with PBS containing 1% BSA as a primary antibody, were added in 100 µl per well and reacted at 37°C for 1 hour. After the reaction, antimouse HRP (Santacruz, sc-516102), which was washed three times and diluted 1,000 times with PBS containing 1% BSA as a secondary antibody, was added 100 µl per well and reacted at 37°C for 1 hour. After the reaction, the reaction mixture was washed three times, and a solution in which Color reagent A and B (R&D systems, DY999) were mixed at a ratio of 1 : 1 was added to 100 µl per well, followed by reaction at room temperature for 15 minutes. Thereafter, 100 µl of 2 N sulfuric acid solution was added to stop the color development reaction, absorbance at 450 nm was measured using a microplate reader, and the degree of binding between each virus and human albumin was compared.

As a result, both pIX-ABD and Hexon-ABD viruses showed significantly higher binding to albumin than the control group, and in particular, Hexon-ABD virus showed significantly higher binding to albumin (FIG. 2).

### Example 3. Compare binding ability of anti-cancer viruses and conventional viruses to human albumin

In order to compare the binding of the anti-cancer virus and human albumin with the existing virus, an immunoprecipitation (IP) analysis was performed. Specifically, 2.0 × 10¹⁰ vp (virus particle) of conventional anti-cancer virus CA10G not including albumin binding domain and anti-cancer virus CA10G-a (HVR1) (Hexon-ABD) including albumin binding domain in the Hexon region were added to tubes, respectively, 1 µg of human albumin was added to each tube, and then the total volume was 1 ml using PBS and incubated at room temperature for 1 hour. Then, 1 µg of anti-albumin antibody and 30 µl of protein A/G agarose were placed in the tube above and stirred at 5 rpm for 4 hours at 4°C, and a sample buffer was used to prepare a Western blot sample. The sample was electrophoresed on 8% SDS-PAGE gel (1.0 mm plate, 10 well comb) at 100 V for 70 minutes, transferred to PVDF membrane, blocked with 5% skim milk, and then incubated at 4°C overnight using Ad5 antibody as a primary antibody. Then, the anti-rabbit antibody was used as a secondary antibody and incubated at room temperature for 1 hour, and the band was confirmed using an HRP substrate.

As a result of checking whether the anti-cancer virus Hexon-ABD (HVR1) of the present invention including the ABD binding sequence is bound to human albumin in a band, unlike CA10G, it was found that the anti-cancer virus bound to albumin (FIG. 3), and thus it is inferred that the anti-cancer virus HVR1 exhibits superior effects than the conventional anti-cancer virus CA10G by avoiding a reaction with a neutralizing antibody when administered in blood.

### Example 4. Check neutralizing antibodies avoidance ability of anti-cancer virus

### 4-1. in vitro

It was confirmed whether the adenovirus including ABD prepared in Example 1 killed cancer cells by avoiding antibody attack by binding to human serum albumin (HSA) *in vivo.* Specifically, the bladder cancer cell line 253JBV, the prostate cancer cell line PC3, and the lung cancer cell line A549 were dispensed into 5, 2.5 × 10³ cells/well, respectively, in a 96-well plate, and then cultured overnight in a CO₂ 5% incubator at 37°C. Thereafter, the ABD-containing adenovirus (Hexon-ABD or pIX-ABD) prepared in the above Example and a control virus (CA10G) not containing ABD were diluted in a medium containing HSA 10 mg/ml (a medium not containing FBS), and then the cells were reacted at room temperature for 30 minutes, and each of the cells was treated with 100 µL/well, Adenovirus type 5 antibody (Abcam, ab6982) was diluted 100 times in a medium not containing FBS, and then the wells treated with HSA + virus were additionally treated with 20 µl each, and then cultured for 72 hours. Thereafter, the degree of virus infection according to the presence or absence of the Adenovirus type 5 antibody was visually confirmed, and the state of the cells by the virus was photographed at 40x magnification, 3 sheets per group. Cell viability was analyzed compared to the control group by measuring the area of cells in photographs taken using the image J program.

As a result, in the case of the bladder cancer cell line, the virus including ABD in pIX or Hexon showed similar apoptosis degree depending on the presence or absence of the antibody, and in particular, Hexon-ABD showed significantly higher ability to kill the cells by avoiding the antibody than pIX-ABD (FIGS. 4 and 5). In addition, it was found that the anti-cancer virus containing ABD in the Hexon of the present invention avoided the antibody and remarkably killed the cells in the prostate cancer cell line and the lung cancer cell line (FIG. 6). Through this, it can be seen that the anticancer adenovirus of the present invention including ABD can kill cancer cells by avoiding the attack of antibodies.

### 4-2. in vivo

Balb/c 6-week-old male mice were injected intraperitoneally (I.P.) with 3.0 × 10¹⁰ vp of wild-type adenovirus (ATCC, VR-1516^{™}), and 7 days later, the same amount of wild-type adenovirus was injected intravenously (I.V). After 7 days, the mouse blood was extracted, centrifuged at 600 g for 5 minutes, and serum of the supernatant was collected, heat-inactivated at 55°C, and stored at -80°C until use. Male Balb/c nu-nu mice aged 6 weeks were implanted with 5.0 × 10⁶ bladder cancer cell line 253J-BV, and their size was observed twice a week, and when the tumor had an average size of 50-100 mm³, the average size of each tumor was regularly divided into groups, and 100 µl of serum was intravenously injected to immunize the mice. After 4 hours of immunization, 3.0 × 10⁹ vp of CA10G and Hexon-ABD (CA10G-a) were injected intratumorally and intravenously, respectively. After 7 days, the mice were euthanized to remove the tumor, the size and weight were confirmed, and immunochemical staining analysis was performed. Specifically, the extracted tissue was molded to the O.C.T. compound and storing it at -80°C for one day, it was sectioned with a thickness of 6 um with a freeze cutter and fixed with 4% PFA. Then, the cells were permeabilized with PBS containing 0.3% triton x-100 for 30 minutes and blocked with PBS containing 5% BSA. Then, the resulting product was reacted with goat anti-Hexon ab (Merck, ab1056) (1 : 200) at room temperature for 1 hour, washed twice with PBS, reacted with anti-goat IgG-conjugated Alexa Fluor 488 antibody (1 : 500) at room temperature for 1 hour, and mounted to analyze images.

The size and weight of tumors were confirmed, and as a result, it was found that CA10G-a anti-cancer virus significantly inhibited the tumor growth, compared to CA10G (FIG. 7). In addition, the results of immunochemical staining analysis also showed that in case of CA10G-a, a larger amount of virus increased even though the same amount of virus was administered (FIG. 7). Through this, it can be confirmed that the anti-cancer virus of the present invention avoids the antibody better than the existing anti-cancer virus by ABD, and thus the tumor growth inhibition function is remarkably increased.

## Claims

1. An anti-tumor adenovirus comprising a nucleic acid encoding an albumin binding domain (ABD).

2. The anti-tumor adenovirus of claim 1, wherein the nucleic acid encoding the albumin binding domain is included in a coding region of Hexon of the adenovirus.

3. The anti-tumor adenovirus of claim 1, wherein the nucleic acid encoding the albumin binding domain is located between codons of a 154th amino acid (gaa, Glu) and a 155th amino acid (gca, Asp) of the Hexon protein encoded by a base sequence represented by SEQ ID NO: 3.

4. The anti-tumor adenovirus of claim 1, wherein the nucleic acid encoding the albumin binding domain is included in a coding region of pIX (protein IX) of the adenovirus.

5. The anti-tumor adenovirus of claim 1, wherein the nucleic acid encoding the albumin binding domain comprises a base sequence represented by SEQ ID NO: 1.

6. The anti-tumor adenovirus of claim 1, wherein the albumin binding domain comprises an amino acid sequence represented by SEQ ID NO: 2.

7. The anti-tumor adenovirus of claim 1, further comprising a human telomere promoter (hTERT).

8. The anti-tumor adenovirus of claim 7, wherein the human telomere promoter is operatively linked to an endogenous gene of the adenovirus.

9. The anti-tumor adenovirus of claim 8, wherein the endogenous gene of the adenovirus has a structure of 5'ITR-C1-C2-C3-C4-C6 3'ITR; wherein the C1 includes E1A, E1B, or E1A-E1B; wherein the C2 includes E2B-L1-L2-L3-E2A-L4; wherein the C3 does not include E3 or includes E3; wherein the C4 includes L5; and wherein the C5 does not include E4 or includes E4.

10. The anti-tumor adenovirus of claim 9, wherein the hTERT promoter is operatively linked to the E1A and the E1B of the endogenous genes of the adenovirus.

11. The anti-tumor adenovirus of claim 9, further comprising an IRES sequence between the E1A and the E1B.

12. The anti-tumor adenovirus of claim 1, further comprising an expression cassette expressing a foreign gene.

13. The anti-tumor adenovirus of claim 12, wherein the expression cassette is included in an E3 region of an endogenous gene of the adenovirus.

14. The anti-tumor adenovirus of claim 1, wherein the anti-tumor adenovirus is selected from the group consisting of human adenovirus serotypes 1 to 57.

15. The anti-tumor adenovirus of claim 1, wherein the anti-tumor adenovirus is human adenovirus serotype 5.

16. The anti-tumor adenovirus of claim 1, wherein the anti-tumor adenovirus has a higher tumor cell killing ability than wild-type adenovirus.

17. The anti-tumor adenovirus of claim 1, wherein the anti-tumor adenovirus is an oncolytic adenovirus.

18. The anti-tumor adenovirus of claim 1, wherein the anti-tumor adenovirus is used for cancer gene therapy.

19. A composition for treating cancer, comprising the anti-tumor adenovirus of claim 1 and a pharmaceutically acceptable carrier.

20. The composition for treating cancer of claim 19, wherein the composition is administered systemically.

21. The composition for treating cancer of claim 19, wherein the composition is for intravenous administration.

22. Use of anti-tumor adenovirus comprising a nucleic acid encoding an albumin binding domain for preventing or treating a tumor.

23. A method for treating a tumor with an anti-tumor adenovirus comprising a nucleic acid encoding an albumin binding domain.
